# EUROPEAN PATENT SPECIFICATION

(11) **EP 1 701 703 B1**
(45) Date of publication and mention of the grant of the patent: **09.03.2011**
(21) Application number: 04806432.3
(22) Date of filing: 20.12.2004
(51) Int. Cl.: A61K 9/16

(54) **STABILIZED PHARMACEUTICAL SOLID COMPOSITIONS OF LOW-SOLUBILITY DRUGS, POLOXAMERS AND STABILIZING POLYMERS**
STABILISIERTE PHARMAZEUTISCHE FESTE ZUSAMMENSETZUNGEN VON ARZNEIMITTELN MIT GERINGER LÖSLICHKEIT, POLOXAMEREN UND STABILISIERENDEN POLYMEREN
COMPOSITIONS PHARMACEUTIQUES SOLIDES STABILISEES DE MEDICAMENTS A FAIBLE SOLUBILITE, POLOXAMERES ET POLYMERES STABILISANTS

(30) Priority: 31.12.2003 US 533848 P
(43) Date of publication of application: 20.09.2006
(73) Proprietor: Bend Research, Inc., Bend, OR 97701-8599 (US)
(72) Inventor: CREW, Marshall David, Bend, OR 97701 (US); SHANKER, Ravi Mysore, Groton, CT 06340 (US); SMITHEY, Daniel Tod, Bend, OR 97701 (US); MILLER, Warren Kenyon, Bend, OR 97701 (US); FRIESEN, Dwayne Thomas, Bend, OR 97702 (US)
(74) Representative: Albrecht, Thomas
(86) International application number: PCT/IB2004/004260
(87) International publication number: WO 2005/065656

(56) References cited:
- EP-A- 1 027 886
- GB-A- 2 394 417
- US-A- 2003 198 674
- B. WOLF: INTERNATIONAL JOURNAL OF PHARMACEUTICS, vol. 156, 1997, pages 97-107, XP002371087

## Description

### FIELD OF THE INVENTION

This invention relates to solid compositions comprising particles comprising a low-solubility drug, a poloxamer, and a stabilizing polymer that provide good physical stability and concentration enhancement of dissolved drug when administered to an aqueous environment of use.

### BACKGROUND OF THE INVENTION

It is sometimes desired to form a composition of amorphous drug and a polymer. One reason for forming such compositions is that the aqueous concentration of a poorly soluble drug may be improved by such a technique. For example, Curatolo, et al., EP 0 901 786 A2 disclose forming pharmaceutical spray dried dispersions of sparingly soluble drugs and the polymer hydroxypropyl methyl cellulose acetate succinate, in which the drug is amorphous and dispersed in the polymer. The spray-dried dispersions disclosed in Curatolo et al. provide superior aqueous concentration relative to dispersions formed from other methods and relative to the crystalline drug alone.

Similarly, others have recognized the enhancement in aqueous concentration afforded by forming compositions of a drug in a polymer. Nakamichi, et al., U.S. Patent No. 5,456,923 disclose solid dispersions formed by twin-screw extrusion of low solubility drugs and various polymers.

Poloxamers (polyoxyethylene-polyoxypropylene copolymers) are routinely used in the pharmaceutical arts for a variety of applications, primarily as emulsifying agents in intravenous fat emulsions, and as solubilizing and stabilizing agents to maintain the clarity of elixirs and syrups. Poloxamers are also used as wetting agents; in ointments, suppository bases, and gels; and as tablet binders and coatings.

Forming compositions of poloxamers and drugs is known. In Xu, et al., Programmable Drug Delivery from an Erodible Association Polymer System, Pharmaceutical Research, Vol. 10, No. 8, pp. 1144-1152 (1993), an erodible association polymer system of Pluronic 127 (a poloxamer) and cellulose acetate phthalate (CAP) was prepared by a solvent casting method. Compositions containing 5% theophylline were prepared. The object of the formulation was to provide a delivery system that provided a programmable dissolved drug concentration versus time profile. Xu, et al., state that for CAP/Pluronic blends in which the CAP content is greater than 50% (w/w), a single miscible phase is formed having only a single glass-transition temperature (T_{g}). Xu et al. measured release rates of the drug from a variety of CAP/Pluronic 127 blends having from 50 to 100 wt% CAP.

Wolf, International Journal of Pharmaceutics 156 (1997), 97-107 discloses compositions of griseofulvin or prednisolone co-precipitated with HPmc and Poloxamer onto a bead cellulose carrier. U.S. Patent No. 6,368,622 to Chen et al. discloses a mixture of drug with a poloxamer. In a particular embodiment, the drug fenofibrate, having a melting point of 72 to 82°C, and a glass-transition temperature of about -19°C, is melted with a poloxamer and mannitol. While the data show the drug in the composition has a faster dissolution rate than a commercial formulation, no concentration enhancement was demonstrated.

U.S. Patent Application Publication No. US2001/0036959A1 to Gabel et al. discloses a composition comprising the drug carvedilol, having a melting point of 113 to 116°C, and a glass-transition temperature of about 39°C, In a concentration above 5 wt%. The preparation preferably includes poloxamers. The composition may be formed using a melt method or by spray drying.

European Patent Specification EP 083847581 to Clancy et al, discloses a solid composition of an active ingredient in a hydrophilic poloxamer polymer. The composition is formed either by melting the poloxamer and dispersing the active ingredient therein or dissolving the active ingredient and poloxamer in an organic solvent or solvents; the solvent is evaporated and the molten poloxamer is cooled and milled to obtain the formulation.

WO 99/21534 discloses a composition comprising a poorly soluble drug and an excipient comprising a mixture of (a) saturated polyglycolized glycerides and (b) polyoxypropylene-polyoxyethylene block copolymers. The compositions are formed by a melt process.

In addition, European Patent Application 1027886A2 notes that dispersions that have improved physical stability can be formed by dispersing an amorphous drug in a polymer with a high T_{g}. However, the concentration enhancement and bioavailability enhancement obtained with such dispersions for some drugs may still be limited and bioavailability may be incomplete-that is, not all of the drug is absorbed.

Forming compositions of amorphous drug and a polymer can present a number of problems. One problem with forming solid compositions containing amorphous drug and a substantial amount of poloxamer is that the drug can crystallize over time, leading to poor performance. Another problem when forming compositions containing an amorphous low-solubility drug is that the drug may have an undesirably slow dissolution rate or poor bioavailability. When absorption of dosed drug is incomplete, that is, the bioavailability is low, drug levels in the blood are often highly variable and exposure is often highly dependent on fed state. Thus, there is a continuing need to provide methods and formulations that both provide amorphous drug that is physically stable and provide enhanced concentrations of drug in aqueous solutions as well as enhanced bioavailability.

### SUMMARY OF THE INVENTION

In a first aspect, a solid composition comprises a plurality of particles, the particles comprising: at least about 5 wt% of a low-solubility drug wherein a substantial portion of the drug is amorphous; at least about 5 wt% of a poloxamer; and a stabilizing polymer. The stabilizing polymer is selected from the group consisting of hydroxypropyl methyl cellulose acetate succinate, and carboxymethyl ethyl cellulose.

The various aspects of the present invention provide one or more of the following advantages. First, the compositions improve the concentration of dissolved drug in an aqueous use environment for poorly soluble drugs. The inventors have found that a problem for some low-solubility drugs dispersed in cellulosic polymers such as hydroxypropyl methyl cellulose acetate succinate or hydroxypropyl methyl cellulose is that the dissolution rate of the drug may be slow. Poloxamers are capable of significantly increasing the dissolution rate of a low-solubility drug, and/or sustaining the concentration of dissolved drug provided by a solid composition containing an amorphous, low-solubility drug. The improved dissolution rate may result in higher dissolved drug concentration, or higher bioavailability, or both.

Second, the addition of a stabilizing polymer improves the physical stability of the particles. Poloxamers are block copolymers consisting of polyethylene oxide (PEO) segments and polypropylene oxide (PPO) segments. Poloxamers have melting points from about 45 to about 60°C. At ambient temperatures typically 10 to 30°C, the PEO segments will eventually aggregate and crystallize to form semicrystalline PEO domains while the PPO segments will remain as amorphous domains. These PPO domains have a relatively low glass-transition temperature (T_{g}), of about -65°C. As a result, any solute dispersed in the amorphous PPO domains will have high mobility at normal storage temperatures of 5 to 40°C. When drug is dispersed in a poloxamer, and subsequently the poloxamer is brought to a temperature below its melt point, the PEO will generally crystallize, and drug will primarily reside in the amorphous PPO domains, where the drug will generally have high mobility. The T_{g} of the drug/PPO domains will generally lie between that of the pure PPO domains and that of pure amorphous drug. The precise value of the T_{g} of such domains will also depend upon the relative amounts of drug and PPO in the domains, and to a lesser extent, the interaction between the drug and the PPO. The inventors have discovered that when the T_{g} of the drug/PPO domains is less than the storage temperature and the concentration of drug in the PPO domains is above its solubility, the drug will have a tendency, over time, to crystallize and the amorphous compositions will therefore be unstable.

Furthermore, the low T_{g} of the particle containing drug and poloxamer makes manufacture of the solid composition more difficult. For example, an efficient and cost-effective method for forming solid compositions containing amorphous drug is via a solvent-based process, such as spray drying. As discussed below, in this process the drug and poloxamer are dissolved in a common solvent to form a solution. This solution is then atomized and the solvent rapidly removed by evaporation. To ensure the solvent is removed at a sufficiently rapid rate to avoid phase separation of the drug and/or polymer, high temperatures may be used. Such high temperatures can make collection of drug/poloxamer particles having low T_{g}s difficult, resulting in low yields and inefficient processes.

To improve the physical stability of particles containing primarily drug and poloxamer, the inventors have found that including a stabilizing polymer in the particles results in compositions having amorphous phases with relatively high T_{g} values, resulting in improved physical stability, as well as increased processing options for formation of the solid compositions. Specifically, the stabilizing polymer is preferably present in a sufficient amount such that the primary drug-containing phase has a T_{g} of least about 40°C, preferably at least about 45°C, and more preferably at least about 50°C when measured at a relative humidity (RH) of less than about 10%. The resulting solid compositions have improved physical stability relative to a control composition consisting essentially of the drug and poloxamer at the same drug loading, but without the stabilizing polymer.

The foregoing and other objectives, features, and advantages of the invention will be more readily understood upon consideration of the following detailed description of the invention.

### DETAILED DESCRIPTION OF THE PREFERRED EMBODIMENTS

The present invention relates to solid compositions comprising particles of a low-solubility drug, a poloxamer, and a stabilizing polymer. The solid compositions of the present invention contain a sufficient amount of poloxamer so as to be capable of achieving high concentrations of dissolved drug in *in vitro* and *in vivo* use environments. The solid compositions contain a stabilizing polymer to provide good physical stability, meaning that the drug in the solid compositions tends to remain in the amorphous form over time at ambient storage conditions. The nature of the solid compositions, suitable poloxamers, stabilizing polymers, and low-solubility drugs, methods for making the compositions, and methods for determining concentration enhancement are discussed in more detail below.

### POLOXAMERS

The drug-containing particles comprise a polyoxyethylene-polyoxypropylene block copolymer, also known in the pharmaceutical arts as a "poloxamer" Poloxamers are crystalline or semicrystalline materials that generally have a molecular weight ranging from about 2000 to about 15,000 daltons and have the general formula:

HO(C₂H₄O)ₐ(C₃H₆O)_{b}(C₂H₄O)ₐH

wherein a is about 10 to about 150, representing blocks of repeat units of polyethylene oxide or polyoxyethylene (referred to herein as the PEO segment), and b is about 20 to about 60, representing blocks of repeat units of polypropylene oxide or polyoxypropylene (referred to herein as the PPO segment), depending on the particular grade. Suitable poloxamers are sold under the trade names PLURONIC and LUTROL available from BASF Corporation of Mt. Olive, New Jersey. Preferred poloxamers have a molecular weight of at least about 4,700 daltons and a melting point of at least about 45°C and so are solid at ambient temperatures.

Preferred grades of poloxamers include poloxamer 188 (PLURONIC F68), poloxamer 237 (PLURONIC F87), poloxamer 338 (PLURONIC F108), poloxamer 407 (PLURONIC F127), the specifications of which are given in Table1, and mixtures of those poloxamers.

**Table 1**

| Poloxamer | Physical Form at 25°C | a | b | Average Molecular Weight (daltons) |
|---|---|---|---|---|
| 188 | Solid | 80 | 27 | 7,680-9,510 |
| 237 | Solid | 64 | 37 | 6,840-8,830 |
| 338 | Solid | 141 | 44 | 12,700-17,400 |
| 407 | Solid | 101 | 56 | 9,840-14,600 |

### STABILIZING POLYMERS

The stabilizing polymer is selected from the group consisting of hydroxypropyl methyl cellulose acetate succinate and carboxymethyl ethyl cellulose. Each of these polymers has a relatively high glass transition temperature relative to the poloxamer. Each of these polymers also has at least some solubility in aqueous solution at physiologically relevant pHs (e.g. 1-8). Of course, when selecting a particular stabilizing polymer, the stabilizing polymer should not chemically react with the drug in an adverse manner.

### SOLID PARTICLES OF DRUG, POLOXAMER AND STABILIZING POLYMER

The solid particles of drug, poloxamer and stabilizing polymer are solid at temperatures up to 30°C and less than 10% relative humidity (RH). In order to keep the total mass of the composition small, it is preferred that the particle comprise at least about 5 wt% drug. More preferably, the particle comprises at least about 10 wt% drug, and more preferably at least about 20 wt% drug. In one embodiment, the particle has a high drug loading. Drug loading refers to the weight fraction of drug in the solid composition. In this embodiment, the drug may be present in an amount of at least about 40 wt% of the particle, at least about 45 wt%, or may even be at least about 50 wt% depending on the glass transition temperature of the particle as discussed below. Such high loadings of drug are desirable to keep the total mass of the pharmaceutical composition at a low value. High drug loadings are possible for physically stable compositions having a high T_{g} (>50°C) as discussed below.

At least a substantial portion of the drug in the particles is amorphous. By "amorphous" is meant that the drug is in a non-crystalline state. As used herein, the term "a substantial portion" of the drug means that at least 75wt% of the drug in the solid particles is in the amorphous form, rather than the crystalline form. It has been found that the aqueous concentration of the drug in a use environmert tends to improve as the fraction of drug present in the amorphous state in the solid composition increases. Accordingly, a "substantial portion" of the drug in the particles is amorphous, and preferably the drug is "almost completely amorphous," meaning that the amount of drug in the crystalline form does not exceed 10 wt%. Amounts of crystalline drug may be measured by powder X-ray diffraction, Scanning Electron Microscope (SEM) analysis, differential scanning calorimetry (DSC), or any other standard quantitative measurement. Most preferably the solid composition is substantially free of crystalline drug.

The amount of poloxamer in the particle may range from at least about 5 wt% to as much as 90 wt% of the particle. The inventors have found that including poloxamers over this broad range in the particles leads to compositions with enhanced dissolution, enhanced bioavailability, or both.

The particles also comprise a stabilizing polymer. As described herein, including a stabilizing polymer in the particles can result in improved physical stability, improved bioavailability, enhanced drug concentrations, or any one or all of these advantages. The amount of stabilizing polymer in the particles will depend on the relative amounts of drug and poloxamer in the particles, as disclosed herein. Generally, the particles comprise from at least about 5 wt% stabilizing polymer to no more than about 90 wt% stabilizing polymer.

The amorphous drug in the solid composition is in intimate contact with the poloxamer and stabilizing polymer. The amorphous drug can exist as a pure phase in the particle, as a solid solution of drug homogeneously distributed throughout the poloxamer and the stabilizing polymer (e.g., a molecular dispersion), or any combination of these states or those states that lie intermediate between them. Without wishing to be bound by any theory, it is believed that the two different block portions of the poloxamer, e.g. the PEO and PPO segments of the poloxamer, are present as different phases in each of the particles. As previously discussed, the PEO portion may be semi-crystalline, such as in the form of lamellar sheets, and as such, contains little if any of the drug. The other phase is composed of amorphous PPO and stabilizing polymer, with all or part of the drug homogeneously dissolved in this phase. In some cases, particularly at high drug loading, a third phase may exist, consisting primarily of amorphous drug. Thus, the drug may be present primarily in the PPO portion, and may be homogeneously distributed throughout the PPO portion and the stabilizing polymer, or the drug may be present as drug-rich domains interspersed throughout the particle, or may be in any combination of these two states or those states that lie intermediate between them. In-cases where drug-rich amorphous domains exist, these domains are generally quite small; that is, less than about 1 µm in size. Preferably, such domains are less than about 100 nm in size. The particles may have a single glass transition temperature, indicating that the drug is homogeneously dispersed throughout the poloxamer and stabilizing polymer, or may have two glass transition temperatures, corresponding to a drug-rich phase and a drug-poor amorphous phase. Thus, while the drug in the particle is amorphous, a portion of the poloxamer in the composition may be in a crystalline or semicrystalline state. Analysis of the solid compositions of the present invention by PXRD or other quantitative methods for determining the crystallinity of a material will typically indicate peaks associated with crystalline or semicrystalline portion of the poloxamer.

Solid compositions of the present invention provide good physical stability. As used herein, "physically stable" or "physical stability" means the tendency of the amorphous drug present in the dispersion to crystallize at ambient storage conditions of 25°C and less than 10% RH. Thus, a solid composition that is more physically stable than another will have a slower rate of drug crystallization in the solid composition. Specifically, compositions of this invention have sufficient stability that less than about 10 wt% of the drug crystallizes during storage for 3 weeks at 25°C and 10% RH. Preferably, less than about 5 wt% of the drug crystallizes during storage for 3 weeks at 25°C and 10% RH, and more preferably, after storage for 3 months at 25°C and 10% RH.

Without wishing to be bound by any particular theory or mechanism of action, it is believed that physically stable particles comprising amorphous drug and polymer generally fall into two categories: (1) those that are thermodynamically stable (in which there is little or no driving force for crystallization of the amorphous drug in the solid composition) and (2) those that are kinetically stable or metastable (in which a driving force exists for crystallization of the amorphous drug but low drug mobility prevents or slows the rate of crystallization to an acceptable level)..

To achieve thermodynamically stable particles, the solubility of the amorphous drug in the polymer should be approximately equal to or greater than the drug loading of the particle. By drug loading is meant the weight fraction of drug in the solid particle. The particles may have a drug loading that is slightly higher than the solubility and still be physically stable, since the driving force for crystal nucleation in that case is quite low. By "slightly higher" is meant a drug loading 10 to 20% higher than the solubility of the drug in the poloxamer. Under these conditions, there is no driving force for crystallization of the amorphous drug. As a result, the particle is thermodynamically stable.

When the drug loading in the solid particles comprising a drug and a polymer is 10% to 20% greater than the solubility of the drug in the polymer (that is, the solid composition is supersaturated in drug), the particles are not thermodynamically stable and a driving force exists for phase separation of the amorphous drug into a drug-rich phase. Such drug-rich phases may be amorphous and microscopic (less than about 1 µm in size), amorphous and relatively large (greater than about 1 µm in size) or crystalline in nature. Thus, following phase separation, the compositions can consist of two or more of the following phases: (1) a drug-rich phase comprising primarily drug; (2) a phase comprising amorphous drug dispersed in the poloxamer and stabilizing polymer; and (3) a phase comprising semicrystalline PEO segments of the poloxamer. The amorphous drug in the drug-rich phases can over time convert from the amorphous form to the lower-energy crystalline form. The physical stability of such particles will generally be greater, for a given drug loading, (1) the lower the molecular mobility of the amorphous drug in the drug-containing phase(s), and (2) the lower the tendency for the amorphous drug to crystallize from the drug-rich phases.

Molecular mobility may be controlled by forming solid particles with high T_{g} values. The T_{g} of the drug-containing phase(s) is an indirect measure of the molecular mobility of the drug in the particles. The ratio of the T_{g} of the drug-containing phase to the storage temperature (T_{storage}) for the solid composition (in K) is an accurate indicator of the relative drug mobility at a given storage temperature. In order to minimize phase separation, it is desired that the mobility of the amorphous drug in the particles is low. This is accomplished by maintaining a ratio of T_{g} of the solid particles/T_{storage} of greater than about 1. Since typical storage temperatures can be as high as 40°C, it is preferred that the T_{g} of the drug-containing phase is at least about 40°C, more preferably at least about 45°C, and most preferably at least about 50°C. Since the T_{g} is a function of the water content of the drug-containing particles, which in turn is a function of the relative humidity to which the drug-containing particles are exposed, these T_{g} values refer to the T_{g} of the particles that have been equilibrated with an atmosphere that has a low relative humidity, that is, less than about 10% of saturation (or an RH of about 10% or less).

The stabilizing polymer is preferably present in the solid compositions of the present invention in a sufficient amount to result in a T_{g} for the primary drugcontaining phase that is at least 40°C, more preferably at least 45°C, and even more preferably at least 50°C. One skilled in the art will realize that the amount of stabilizing polymer needed to achieve this will be dependent on the amount and properties of the drug and poloxamer present. In a preferred embodiment, the stabilizing polymer is present in a sufficient amount such that the lowest T_{g} of the particle is at least 40°C, more preferably at least 45°C and even more preferably at least 50°C.

In one embodiment, the poloxamer is the primary non-drug component of the particles. In this embodiment, the mass ratio of poloxamer to stabilizing polymer in the solid composition is greater than about 1, may be greater than about 1.5, or may be greater than about 2. The particle may comprise from about 5 wt% to about 40 wt% stabilizing polymer. Depending on the properties of the drug, including small amounts of a stabilizing polymer in the solid composition, such as at least 5wt%, 10 wt%, or 20 wt%, can raise the T_{g} of the particles sufficiently to result in improved physical stability.

This embodiment, in which the poloxamer is the primary component of the particle, has particular utility for drugs that have a glass transition temperature that is close to or greater than ambient storage conditions. Without wishing to be bound by any particular theory, it is believed that the higher the T_{g} of the drug, the higher the Tg of the particle, and the lower the molecular mobility of the amorphous drug in the solid particle. As a result, solid particles formed with low-solubility drugs having moderate to high T_{g} values and a significant amount of poloxamer tend to have higher T_{g} values themselves, and as a result, require smaller amounts of a stabilizing polymer to improve the physical stability of the amorphous drug. Thus, the T_{g} of the drug alone in this embodiment may be at least about 20°C, but preferably is at least about 30°C, and even more preferably is at least about 40°C. (Unless otherwise noted, asused herein the T_{g} refers to the T_{g} measured at a relative humidity of less than 10%.) The T_{g} of a drug can be determined using standard analytical techniques well known in the art, including by a dynamic mechanical analyzer (DMA), a dilatometer, a dielectric analyzer, and by differential scanning calorimetry (DSC).

In a separate embodiment, the stabilizing polymer constitutes the primary non-drug component of the particles. This embodiment has particular utility for drugs that have either low glass transition temperatures, or that have an undesirably low dissolution rate or poor bioavailability. In the first case, particles containing a drug with a glass transition temperature of less than 40°C may nonetheless have a glass transition temperature of the particle above 40°C by including a significant amount of stabilizing polymer. This embodiment also has utility for drugs that may have a high glass transition temperature but that have an undesirably low dissolution rate, poor bioavailability, or both. The addition of a relatively small amount of poloxamer to a molecular dispersion consisting primarily of the amorphous drug and stabilizing polymer may result in significant improvement in the dissolution rate, or bioavailability, or both. In this embodiment, the mass ratio of stabilizing polymer to poloxamer in the solid composition is greater than about 1, may be greater than about 1.5, or may be greater than about 2. The particle may comprise from about 5 wt% to about 40 wt% poloxamer. Depending on the properties of the drug, including small amounts of a poloxamer in the particle, such as at least 5wt%, 10 wt%, or 20 wt% of the particle, can improve the dissolution rate, improve bioavailability, or both.

### LOW-SOLUBILITY DRUGS

The term "drug" is conventional, denoting a compound having beneficial prophylactic and/or therapeutic properties when administered to an animal, especially humans. Preferably, the drug is a "low-solubility drug," meaning that the drug has a minimum aqueous solubility at physiologically relevant pH (e.g., pH 1-8) of about 0.5 mg/mL or less. The invention finds greater utility as the aqueous solubility of the drug decreases. Thus, compositions of the present invention are preferred for low-solubility drugs having an aqueous solubility of less than about 0.1 mg/mL, more preferred for low-solubility drugs having an aqueous solubility of less than about 0.05 mg/mL, and even more preferred for low-solubility drugs having an aqueous solubility of less than about 0.01 mg/mL. In general, it may be said that the drug has a dose-to-aqueous solubility ratio greater than about 10 mL, and more typically greater than about 100 mL, where the aqueous solubility (mg/mL) is the minimum value observed in any physiologically relevant aqueous solution (e.g., those with pH values between 1 and 8) including USP simulated gastric and intestinal buffers, and dose is in mg. Thus, a dose-to-aqueous solubility ratio may be calculated by dividing the dose (in mg) by the aqueous solubility (in mg/mL).

Preferred classes of drugs include, but are not limited to, antihypertensives, antianxiety agents, anticlotting agents, anticonvulsants, blood glucose-lowering agents, decongestants, antihistamines, antitussives, antineoplastics, beta blockers, anti-inflammatories, antipsychotic agents, cognitive enhancers, cholesterol-reducing agents, anti-atherosclerotic agents, antiobesity agents, autoimmune disorder agents, anti-impotence agents, antibacterial and antifungal agents, hypnotic agents, anti-Parkinsonism agents, anti-Alzheimer's disease agents, antibiotics, anti-depressants, antiviral agents, glycogen phosphorylase inhibitors, and cholesteryl ester transfer protein (CETP) inhibitors.

Each named drug should be understood to include any pharmaceutically acceptable forms of the drug. By "pharmaceutically acceptable forms" is meant any pharmaceutically acceptable derivative or variation, including stereoisomers, stereoisomer mixtures, enantiomers, solvates, hydrates, isomorphs, polymorphs, pseudomorphs, neutral forms, salt forms and prodrugs. Specific examples of antihypertensives include prazosin, nifedipine, amiodipine besylate, trimazosin and doxazosin; specific examples of a blood glucose-lowering agent are glipizide and chlorpropamide; a specific example of an anti-impotence agent is sildenafil and sildenafil citrate; specific examples of antineoplastics include chlorambucil, lomustine and echinomycin; a specific example of an imidazole-type antineoplastic is tubulazole; a specific example of an anti-hypercholesterolemic is atorvastatin calcium; specific examples of anxiolytics include hydroxyzine hydrochloride and doxepin hydrochloride; specific examples of anti-inflammatory agents include betamethasone, prednisolone, aspirin, piroxicam, valdecoxib, carprofen, celecoxib, flurbiprofen and (+)-N-{4-[3-(4-fluorophenoxy)phenoxy]-2-cyclopenten-1-yl}-N-hyroxyurea; a specific example of a barbiturate is phenobarbital; specific examples of antivirals include acyclovir, nelfinavir, and virazole; specific examples of vitamins/nutritional agents include retinol and vitamin E; specific examples of beta blockers include timolol and nadolol; a specific example of an emetic is apomorphine; specific examples of a diuretic include chlorthalidone and spironolactone; a specific example of an anticoagulant is dicumarol; specific examples of cardiotonics include digoxin and digitoxin; specific examples of androgens include 17-methyltestosterone and testosterone; a specific example of a mineral corticoid is desoxycorticosterone; a specific example of a steroidal hypnotic/anesthetic is alfaxalone; specific examples of anabolic agents include fluoxymesterone and methanstenolone; specific examples of antidepression agents include sulpiride, [3,6-dimethyl-2-(2,4,6-trimethyl-phenoxy)-pyridin-4-yl]-(1-ethylpropyl)-amine,3,5-dimethyl-4-(3'-pentoxy)-2-(2',4',6'-trimethylphenoxy)pyridine, pyroxidine, fluoxetine, paroxetine, venlafaxine and sertraline; specific examples of antibiotics include carbenicillin indanylsodium, bacampicillin hydrochloride, troleandomycin, doxycyline hyclate, ampicillin and penicillin G; specific examples of anti-infectives include benzalkonium chloride and chlorhexidine; specific examples of coronary vasodilators include nitroglycerin and mioflazine; a specific example of a hypnotic is etomidate; specific examples of carbonic anhydrase inhibitors include acetazolamide and chlorzolamide; specific examples of antifungals include econazole, terconazole, fluconazole, voriconazole, and griseofulvin; a specific example of an antiprotozoal is metronidazole; specific examples of anthelmintic agents include thiabendazole and oxfendazole and morantel; specific examples of antihistamines include astemizole, levocabastine, cetirizine, decarboethoxyloratadine and cinnarizine; specific examples of antipsychotics include ziprasidone, olanzepine, thiothixene hydrochloride, fluspirilene, risperidone and penfluridole; specific examples of gastrointestinal agents include loperamide and cisapride; specific examples of serotonin antagonists include ketanserin and mianserin; a specific example of an anesthetic is lidocaine; a specific example of a hypoglycemic agent is acetohexamide; a specific example of an anti-emetic is dimenhydrinate; a specific example of an antibacterial is cotrimoxazole; a specific example of a dopaminergic agent is L-DOPA; specific examples of anti-Alzheimer's Disease agents are THA and donepezil; a specific example of an anti-ulcer agent/H2 antagonist is famotidine; specific examples of sedative/hypnotic agents include chlordiazepoxide and triazolam; a specific example of a vasodilator is alprostadil; a specific example of a platelet inhibitor is prostacyclin; specific examples of ACE inhibitor/antihypertensive agents include enalaprilic acid and lisinopril; specific examples of tetracycline antibiotics include oxytetracycline and minocycline; specific examples of macrolide antibiotics include erythromycin, clarithromycin, and spiramycin; a specific example of an azalide antibiotic is azithromycin; specific examples of glycogen phosphorylase inhibitors include [R-(R*S*)]-5-chloro-N-[2-hydroxy-3-(methoxymethylamino)-3-oxo-1-(phenylmethyl)propyl-1H-indole-2-carboxamide and 5-chloro-1H-indole-2-carboxylic acid [(1S)-benzyl-(2R)-hydroxy-3-((3R,4S)-dihydroxy-pyrrolidin-1-yl-)-3-oxypropyl]amide; and specific examples of cholesteryl ester transfer protein (CETP) Inhibitors Include [2R,45] 4[(3,5-bis-trifluoromethyl-benzyl)-methoxycarbonyl-amino]2-ethyl-6-trifluoromethyl-3,4-dihydro-2H-quinolin-1-carboxylic acid ethyl ester, [2R,4S] 4-[acetyl-(3,5-bis-trifluoromethyl-benzyl)-amino]-2-ethyl-6-trifluoromethyl-3,4-dihydro-2H-quinoline-1 -carboxylic acid isopropyl ester, [2R, 4S] 4-[(3,5-bis-trifluoromethyl-benzyl)-methyloxycarbonyl-amino]-2-ethyl-6-trifluoromethyl-3,4-dihydro-2H-quinoline-1 -carboxylic acid isopropyl ester, (2R)-3-[[3-(4-chloro-3-ethylphenoxy)phenyl][3-(1,1,2,2-tetrafluoroethoxy)phenyl]methyl]amino]-1,1,1-trüfluoro-2-propanol, the drugs disclosed in commonly owned U.S. Patent Application Serial Nos. 09/916.127 and 10/066.091 and the drugs disclosed In the following patents and published applications: DE 19741400 A1; DE 19741399 A1; WO 9914215 A1; WO 9914174; DE 19708125 A1; DE 19704244 A1: DE 19704243 A1: EP 818448 A1; WO 9804628 A2. DE 19627431 A1; DE 19627430 A1; DE 19627419 A1; EP 796846 A1; DE 19832159; DE 818197; DE 19741051; WO 9941237 A1; WO 9914204 A1; WO 9835937 A1; JP 11049743; WO 200018721; WO 200018723; WO 200018724; WO 200017164; WO 200017165: WO 200017166: EP 992496; and EP 987251.

The present invention has particular utility for use with hydrophobic drugs. In contrast to conventional wisdom, the relative degree of enhancement In aqueous concentration and bioavailability provided by the compositions of the present invention generally improves for drugs as solubility decreases and hydrophobicity increases. In fact, the inventors have recognized a subclass of hydrophobic drugs that are essentially aqueous insoluble, highly hydrophobic, and are characterized by a set of physical properties. This subclass; referred to herein as "hydrophobic drugs," exhibits dramatic enhancements in aqueous concentration and bioavailability when formulated using the polymers of the present invention.

The first property of hydrophobic drugs is extremely low aqueous solubility. By extremely low aqueous solubility is meant that the minimum aqueous solubility at physiologically relevant pH (pH of 1 to 8) is less than about 10µg/ml and typically less than about 1 µg/ml.

A second property is a very high dose-to-solubility ratio. Extremely low aqueous solubility often leads to poor or slow absorption of the drug from the fluid of the gastrointestinal tract, when the drug is dosed orally in a conventional manner. For extremely low solubility drugs, poor absorption generally becomes progressivity more difficult as the dose (mass of drug given orally) increases. Thus, a second property of hydrophobic drugs is a very high dose (in mg) to solubility (in mg/ml) ratio (ml). By "very high dose-to-solubility ratio" is meant that the dose-to-solubility ratio may have a value of at least 1000 ml, at least 5,000ml, or even at least 10,000ml.

A third property of hydrophobic drugs is that they are extremely hydrophobic. By extremely hydrophobic is meant that the Log P value of the drug may have a value of at least 4.0, a value of at least 5.0, and even a value of at least 5.5. Log P, defined as the base 10 logarithm of the ratio of the drug solubility in octanol to the drug solubility in water, is a widely accepted measure of hydrophobicity. Log P may be measured experimentally or calculated using methods known in the art. Calculated Log P values are often referred to by the calculation method, such as Clog P, Alog P, and Mlog P.

A fourth property of hydrophobic drugs is that they have a low melting point. Generally, drugs of this subclass will have a melting point of about 150°C or less, and often about 140°C or less.

Primarily, as a consequence of some or all of these four properties, hydrophobic drugs typically have very low absolute bioavailabilities. Specifically, the absolute bioavailability of drugs in this subclass when dosed orally in their undispersed state is less than about 10% and more often less than about 5%. The use of a poloxamer in combination with such drugs may improve their dissolution rate, bioavailability, or both. Hydrophobic drugs tend to have slow dissolution rates and low absolute bioavailability. The poloxamer may act as a dissolution enhancer to increase the dissolution rate of the drug. In addition, when the solid compositions are introduced to an aqueous environment of use, the hydrophobic drugs partition into the poloxamer micelles that form in the aqueous environment, increasing the concentration of dissolved drug, as discussed further herein. The stabilizing polymers function to improve the stability of the poloxamer/drug system. As a result, solid compositions comprising hydrophobic drugs, poloxamers, and stabilizing polymers provide a unique combination of physical stability and concentration enhancement.

### METHODS FOR MAKING PARTICLES CONTAINING DRUG, POLOXAMER AND STABILIZING POLYMER

The particles containing drug, poloxamer, and stabilizing polymer may be formed by any conventional method, such as by solvent processes, thermal process, or mechanical processes, that results in at least a substantial portion (that is, at least 75 wt%) of the drug being in the amorphous state.

The particles of drug, poloxamer, and stabilizing polymer are well suited for formation via solvent processes. The high T_{g} of the solid compositions of the present invention allow for selection of processing conditions that lead to formation of solid materials with a minimum of phase separation of the drug in the particles. By phase separation is meant that a significant amount of the drug in the particles separates into domains rich in amorphous drug. When phase separation does occur and drug-rich domains form, choosing conditions where solvent is removed rapidly causes the domains to be quite small-generally less than about 1 µm in size and preferably less than about 200 nm in size.

In solvent processes, the low-solubility drug, poloxamer, and stabilizing polymer are dissolved in a common solvent. "Common" here means that the solvent, which can be a mixture of compounds, will dissolve the drug, the poloxamer, and the stabilizing polymer. After the drug, poloxamer, and stabilizing polymer have been dissolved, the solvent is removed by evaporation or by mixing with a non-solvent. Exemplary processes are spray-drying, spray-coating (pan-coating, fluidized bed coating, etc.), rotoevaporation, and precipitation by rapid mixing of the polymer and drug solution with CO₂, water, or some other non-solvent. Preferably, removal of the solvent results in at least a substantial portion of the drug being in the amorphous state.

Solvents suitable for solvent processing are preferably volatile with a boiling point of 150°C or less. In addition, the solvent should have relatively low toxicity and be removed from the solid composition to a level that is acceptable according to The International Committee on Harmonization (ICH) guidelines. Removal of solvent to this level may require a subsequent processing step such as tray drying. Preferred solvents include water; alcohols such as methanol, and ethanol; ketones such as acetone, methyl ethyl ketone and methyl iso-butyl ketone; and various other solvents such as acetonitrile, methylene chloride, and tetrahydrofuran. Lower volatility solvents such as dimethyl acetamide or dimethylsulfoxide can also be used in small amounts in mixtures with a volatile solvent. Mixtures of solvents, such as 50% methanol and 50% acetone, can also be used, as can mixtures with water, so long as the poloxamer, stabilizing polymer, and drug are sufficiently soluble to make the process practicable. Generally, due to the hydrophobic nature of low-solubility drugs, non-aqueous solvents are preferred, meaning that the solvent comprises less than about 30 wt% water.

The solvent may be removed by spray-drying. The term "spray-drying" is used conventionally and broadly refers to processes involving breaking up liquid mixtures Into small droplets (atomization) and rapidly removing solvent from the mixture in a spray-drying apparatus where there is a strong driving force for evaporation of solvent from the droplets. Spray-drying processes and spray-drying equipment are described generally in Perry's Chemical Engineers' Handbook, pages 20-54 to 20-57 (Sixth Edition 1984). More details on spray-drying processes and equipment are reviewed by Marshall, "Atomization and Spray-Drying," 50 Chem. Eng. Prog. Monogr. Series 2 (1954), and Masters, Spray Drying Handbook (Fourth Edition 1985). The strong driving force for solvent evaporation is generally provided by maintaining the partial pressure of solvent in the spray-drying apparatus well below the vapor pressure of the solvent at the temperature of the drying droplets. This is accomplished by (1) maintaining the pressure in the spray-drying apparatus at a partial vacuum (e.g., 0.01 to 0,50 atm); or (2) mixing the liquid droplets with a warm drying gas; or (3) both (1) and (2). In addition, at least a portion of the heat required for evaporation of solvent may be provided by heating the spray solution.

The solvent-bearing feed, comprising the drug, the poloxamer, and the stabilizing polymer can be spray-dried under a wide variety of conditions and yet still yield compositions with acceptable properties. For example, various types of nozzles can be used to atomize the spray solution, thereby introducing the spray solution into the spray-dry chamber as a collection of small droplets. Essentially any type of nozzle may be used to spray the solution as long as the droplets that are formed are sufficiently small that they dry sufficiently (due to evaporation of solvent) that they do not stick to or coat the spray-drying chamber wall.

Although the maximum droplet size varies widely as a function of the size, shape and flow pattern within the spray-dryer, generally droplets should be less than about 500 µm in diameter when they exit the nozzle. Examples of types of nozzles that may be used to form the solid compositions include the two-fluid nozzle, the fountain-type nozzle, the flat fan-type nozzle, the pressure nozzle and the rotary atomizer. In a preferred embodiment, a pressure nozzle is used, as disclosed In detail in commonly assigned copending U.S. Application No. 10/351,568.

The spray solution can be delivered to the spray nozzle or nozzles at a wide range of temperatures and flow rates. Generally, the spray solution temperature can range anywhere from Just above the solvent's freezing point to about 20°C above its ambient pressure boiling point (by pressurizing the solution) and in some cases even higher. Spray solution flow rates to the spray nozzle can vary over a wide range depending on the type of nozzle, spray-dryer size and spray-dry conditions such as the inlet temperature and flow rate of the drying gas. Generally, the energy for evaporation of solvent from the spray solution in a spray-drying process comes primarily from the drying gas.

The drying gas can, in principle, be essentially any gas, but for safety reasons and to minimize undesirable oxidation of the drug or other materials In the solid composition, an inert gas such as nitrogen, nitrogen-enriched air or argon is utilized. The drying gas is typically introduced into the drying chamber at a temperature between about 60° and about 300°C and preferably between about 80° and about 240°C.

The large surface-to-volume ratio of the droplets and the large driving force for evaporation of solvent leads to rapid solidification times for the droplets. Solidification times should be less than about 20 seconds, preferably less than about 10 seconds, and more preferably less than 1 second. This rapid solidification is often critical to the particles maintaining a uniform, homogeneous phase of drug and polymer instead of separating into drug-rich and polymer-rich phases. In a preferred embodiment, the height and volume of the spray-dryer are adjusted to provide sufficient time for the droplets to dry prior to impinging on an internal surface of the spray-dryer, as described in detail in U.S. Patent No. 6,763,607.

The inventors have found that while the properties of the spray dried composition can vary greatly depending on the spray drying conditions, nevertheless the temperature of the exhaust drying gas at the outlet, or T_{OUT}, appears to be critical to producing solid particles with a minimum of phase separation of the drug. Generally, it is desirable that the solvent be rapidly removed, resulting in rapid solidification of the droplets. In conventional spray-drying applications, this is accomplished by raising T_{OUT,} However, If T_{OUT} Is greater than the glass transition temperature of the particles, the mobility of the drug in the particles is high during the spray-drying process, and the drug may phase separate in the composition, and may ultimately crystallize. Thus, particles having a minimum amount of phase separated drug are most likely to result when T_{OUT} is maintained below the glass transition temperature of the solid composition. Preferably, T_{OUT} is less than the T_{g} of the solid particles plus 20°C (T_{g} + 20°C), and preferably less than the T_{g}. The higher the T_{g} of the composition, the higher T_{OUT} can be while producing particles having minimum amounts of phase separated drug.

Following solidification, the solid powder typically stays in the spray-drying chamber for about 5 to 60 seconds, further evaporating solvent from the solid powder. The final solvent content of the solid composition as it exits the dryer should be low, since this reduces the mobility of the drug molecules in the solid composition, thereby improving its stability. Generally, the solvent content of the solid composition as it leaves the spray-drying chamber should be less than 10wt% and preferably less than 2 wt%. Following formation, the solid composition can be dried to remove residual solvent using suitable drying processes, such as tray drying, fluid bed drying, microwave drying, belt drying, rotary drying, vacuum drying, and other drying processes known in the art.

Spray-drying processes and spray-drying equipment are described generally in Perry's Chemical Engineers' Handbook, Sixth Edition (R. H. Perry, D. W. Green, J. O. Maloney, eds.) McGraw-Hill Book Co. 1984, pages 20-54 to 20-57. More details on spray-drying processes and equipment are reviewed by Marshall "Atomization and Spray-Drying," 50 Chem. Eng. Prog. Monogr. Series 2 (1954).

In another embodiment, the solid composition is formed by a rotoevaporation process. In this process the drug, poloxamer, and stabilizing polymer are dissolved in a common solvent as described above. The solvent is then removed by rotoevaporation. The resulting thick syrup or solids may then be dried on a high vacuum line. The resulting solids are preferably formed into small particles, such as by using a mortar and pestle or other milling processes known in the art. The particles may be sieved and dried as necessary to obtain a material with the desired properties.

In another embodiment, the particles are formed by spraying a coating solution of the drug, poloxamer and stabilizing polymer onto seed cores. The seed cores can be made from any suitable material such as starch, microcrystalline cellulose, sugar or wax, by any known method, such as melt- or spray-congealing, extrusion/spheronization, granulation, spray-drying and the like.

The coating solution can be sprayed onto such seed cores using coating equipment known in the pharmaceutical arts, such as pan coaters (e.g., Hi-Coater available from Freund Corp. of Tokyo, Japan, Accela-Cota available from Manesty of Liverpool, U.K.), fluidized bed coaters (e.g., Würster coaters or top-sprayers available from Glatt Air Technologies of Ramsey, New Jersey and from Niro Pharma Systems of Bubendorf, Switzerland) and rotary granulators (e.g., CF-Granulator, available from Freund Corp).

### CONCENTRATION ENHANCEMENT

The solid compositions of the present invention are concentration enhancing. The term "concentration enhancing" means that the poloxamer is present in a sufficient amount in the composition so as to improve the concentration of the drug in a use environment relative to a control composition. As used herein, a "use environment" can be either the *in vivo* environment of the GI tract, subdermal, intranasal, buccal, intrathecal, ocular, intraaural, subcutaneous spaces, vaginal tract, arterial and venous blood vessels, pulmonary tract or intramuscular tissue of an animal, such as a mammal and particularly a human, or the *in vitro* environment of a test solution, such as phosphate buffered saline (PBS) or a Model Fasted Duodenal (MFD) solution. Concentration enhancement may be determined through either *in vitro* dissolution tests or through *in vivo* tests. It has been determined that enhanced drug concentration in *in vitro* dissolution tests in Model Fasted Duodenal (MFD) solution or Phosphate Buffered Saline (PBS) is a good indicator of *in vivo* performance and bioavailability. An appropriate PBS solution is an aqueous solution comprising 20 mM sodium phosphate (Na₂HPO₄), 47 mM potassium phosphate (KH₂PO₄), 87 mM NaCl, and 0.2 mM KCI, adjusted to pH 6.5 with NaOH. An appropriate MFD solution is the same PBS solution wherein additionally is present 7.3 mM sodium taurocholic acid and 1.4 mM of 1-palmitoyl-2-oleyl-sn-glycero-3-phosphocholine. In particular, a composition of the present invention may be dissolution-tested by adding it to MFD or PBS solution and agitating to promote dissolution.

In one aspect, a composition of the present invention, when dosed to an aqueous use environment, provides a maximum drug concentration (MDC) that is at least 1.25-fold that of a control composition. In other words, if the MDC provided by the control composition is 100 µg/mL, then a composition of the present invention provides an MDC of at least 125 µg/mL. More preferably, the MDC of drug provided by a composition of the present invention is at least 2-fold, even more preferably at least 3-fold, and most preferably at least 5-fold that of the control composition.

The control composition is conventionally the undispersed drug alone (e.g., typically, the crystalline drug alone in its most thermodynamically stable crystalline form, or in cases where a crystalline form of the drug is unknown, the control may be the amorphous drug alone) or the drug plus a weight of inert diluent equivalent to the weight of poloxamer and stabilizing polymer in the test composition. By inert is meant that the diluent is not concentration enhancing.

Preferably, the solid compositions provide concentration enhancement relative to a control composition consisting essentially of a particle consisting of an equivalent drug loading of amorphous drug in the stabilizing polymer but no poloxamer.

Alternatively, the compositions of the present invention provide in an aqueous use environment a concentration versus time Area Under the Curve (AUC), for any period of at least 90 minutes between the time of introduction into the use environment and about 270 minutes following introduction to the use environment that is at least 1.25-fold that of the control-composition. More preferably, the AUC in the aqueous use environment provided by the compositions of the present invention are at least 2-fold, more preferably at least 3-fold, and most preferably at least 5-fold that of a control composition.

Alternatively, the compositions of the present invention, when dosed orally to a human or other animal, provide an AUC in drug concentration in the blood plasma or serum that is at least 1.25-fold that observed when an appropriate control composition is dosed. Preferably, the blood AUC is at least about 2-fold, preferably at least about 3-fold, preferably at least about 4-fold, preferably at least about 6-fold, preferably at least about 10-fold, and even more preferably at least about 20-fold that of the control composition. It is noted that such compositions can also be said to have a relative bioavailability of from about 1.25-fold to about 20-fold that of the control composition. Thus, the compositions that, when evaluated, meet either the *in vitro* or the *in vivo,* or both, performance criteria are a part of this invention.

Alternatively, the compositions of the present invention, when dosed orally to a human or other animal, provide a maximum drug concentration in the blood plasma or serum (Cₘₐₓ) that is at least 1.25-fold that observed when an appropriate control composition is dosed. Preferably, the blood Cₘₐₓ is at least about 2fold, preferably at least about 3-fold, preferably at least about 4-fold, preferably at least about 6-fold, preferably at least about 10-fold, and even more preferably at least about 20-fold that of the control composition.

A typical *in vitro* test to evaluate enhanced drug concentration can be conducted by (1) administering with agitation a sufficient quantity of test composition (that is, the composition of the low-solubility drug, poloxamer, and stabilizing polymer) in a test medium, such that if all of the drug dissolved, the theoretical concentration of drug would exceed the equilibrium concentration of the drug by a factor of at least 2; (2) in a separate test, adding an appropriate amount of control composition to an equivalent amount of test medium; and (3) determining whether the measured MDC and/or AUC of the test composition in the test medium is at least 1.25-fold that provided by the control composition. In conducting such a dissolution test, the amount of test composition or control composition used is an amount such that if all of the drug dissolved, the drug concentration would be at least 2-fold, preferably at least 1 0-fold, and most preferably at least 100-fold that of the solubility (that is, the equilibrium concentration), of the drug. For some test compositions of a very low-solubility drug, a poloxamer, and a stabilizing polymer, it may be necessary to administer an even greater amount of the test composition to determine the MDC.

The concentration of dissolved drug is typically measured as a function of time by sampling the test medium and plotting drug concentration in the test medium vs. time so that the MDC and/or AUC can be ascertained. The MDC is taken to be the maximum value of dissolved drug measured over the duration of the test. The aqueous AUC is calculated by integrating the concentration versus time curve over any 90-minute time period between the time of introduction of the composition into the aqueous use environment (when time equals zero) and 270 minutes following introduction to the use environment (when time equals 270 minutes). Typically, when the composition reaches its MDC rapidly, in say less than about 30 minutes, the time interval used to calculate AUC is from time equals zero to time equals 90minutes. However, if the AUC of a composition over any 90-minute time period described above meets the criterion of this invention, then the composition formed is considered to be within the scope of this invention.

To avoid drug particulates that would give an erroneous determination, the test solution is either filtered or centrifuged. "Dissolved drug" is typically taken as that material that either passes a 0.45 µm syringe filter or, alternatively, the material that remains in the supernatant following centrifugation. Filtration can be conducted using a 13 mm, 0.45 µm polyvinylidine difluoride syringe filter sold by Scientific Resources under the trademark TITAN®. Centrifugation is typically carried out in a polypropylene microcentrifuge tube by centrifuging at 13,000 G for 60seconds. Other similar filtration or centrifugation methods can be employed and useful results obtained. For example, using other types of microfilters may yield values somewhat higher or lower (±10-40%) than that obtained with the filter specified above but will still allow identification of preferred compositions. It is recognized that this definition of "dissolved drug" encompasses not only monomeric solvated drug molecules but also a wide range of species such as polymer/drug assemblies that have submicron dimensions such as drug aggregates, aggregates of mixtures of polymer and drug, micelles, polymeric micelles, colloidal particles or nanocrystals, polymer/drug complexes, and other such drug-containing species that are present in the filtrate or supernatant in the specified dissolution test.

Alternatively, the compositions of the present invention, when dosed orally to a human or other animal, results in improved bioavailability or an enhanced Cₘₐₓ. The relative bioavailability and Cₘₐₓ of drugs in the compositions can be tested *in vivo* in animals or humans using conventional methods for making such a determination. An *in vivo* test, such as a crossover study, may be used to determine whether a composition of drug and poloxamer provides an enhanced relative bioavailability or Cₘₐₓ compared with a control composition as described above. In an *in vivo* crossover study a test composition comprising a low-solubility drug, a poloxamer, and a stabilizing polymer is dosed to half a group of test subjects and, after an appropriate washout period (e.g., one week) the same subjects are dosed with a control composition described above. The other half of the group is dosed with the control composition first, followed by the test composition. The relative bioavailability is measured as the concentration of drug in the blood (serum or plasma) versus time area under the curve (AUC) determined for the test group divided by the AUC in the blood provided by the control composition. Preferably, this test/control ratio is determined for each subject, and then the ratios are averaged over all subjects in the study. Likewise, the Cₘₐₓ may be determined from the concentration of drug in the blood versus time for the test group divided by that provided by the control composition. *In vivo* determinations of Cₘₐₓ and AUC can be made by plotting the serum or plasma concentration of drug along the ordinate (y-axis) against time along the abscissa (x-axis). To facilitate dosing, a dosing vehicle may be used to administer the dose. The dosing vehicle is preferably water, but may also contain materials for suspending the test or control composition, provided these materials do not dissolve the composition or change the drug solubility *in vivo.* The determination of Cₘₐₓ and AUCs is a well-known procedure and is described, for example, in Welling, "Pharmacokinetics Processes and Mathematics," ACS Monograph 185 (1986).

### IMPROVED PHYSICAL STABILITY

In one embodiment, the solid particles of drug, poloxamer, and stabilizing polymer provide improved physical stability relative to a control composition. For determining improvements in physical stability, the control composition consists essentially of particles having the same drug loading in poloxamer only, but without the stabilizing polymer.

An improvement in physical stability may be determined by comparing the rate of crystallization of the drug in a "test composition" comprising a drug, poloxamer, and stabilizing polymer of the present invention, with the rate of crystallization of drug in the control composition. The rate of crystallization of drug may be measured by determining the fraction of drug in the crystalline state in the test composition or control composition over time in a typical storage environment. This may be measured by any standard physical measurement, such as x-ray diffraction, DSC, solid state NMR or Scanning Electron Microscope ("SEM") analysis. Drug in a physically stable test composition will crystallize at a slower rate than the drug in the control composition. Preferably, the rate of crystallization of the drug in the test composition is less than 90%, and more preferably less than 80%, of the rate of crystallization of drug in the control composition. Thus, for example, if the drug in the control composition crystallizes at a rate of 1 %/week, the drug in the test composition crystallizes at a rate of less than 0.9%/week. Often, much more dramatic improvements are observed, such as less than about 10% of the rate of crystallization of drug in the control composition (or less than about 0.1 %/week for the example given).

A relative degree of improvement in physical stability may be used to characterize the improvement in physical stability obtained by the compositions of the present invention. The "relative degree of improvement in physical stability" is defined as the ratio of (1) the rate of drug crystallization in the control composition and (2) the rate of drug crystallization in the test composition. For example, if the drug in the control composition crystallizes at a rate of 10 wt%/week and the drug in the test composition crystallizes at a rate of 5 wt%/week, the relative degree of improvement in physical stability would be 2 (10 wt%/week ÷5 wt%/week). Preferably, the compositions of the present invention provide a relative degree of improvement in physical stability of at least about 1.25, preferably at least about 2.0, more preferably at least about 3.0 relative to a control composition consisting essentially of the same amounts of drug and poloxamer, but without the stabilizing polymer.

The particular storage conditions and time of storage to evaluate physical stability may be chosen as convenient. A stability test which may be used to test whether a composition meets the stability criteria described above is storage of the test composition and the control composition for three weeks at 25°C and 10% RH. An improvement of stability for the test composition may become apparent within a shorter time, such as three to five days, and shorter storage times may be used for some drugs. The stability test may also be accelerated by performing the test at elevated temperature and humidity, such as for 1 to 3 weeks at 40°C and 25% RH, or at 40°C and 75% RH.

### EXCIPIENTS AND DOSAGE FORMS

Other conventional formulation excipients may be employed in the compositions of this invention, including those excipients well-known in the art (*e.g*, as described in Remington: The Science and Practice of Pharmacy (20th ed., 2000)). Generally, excipients such as fillers, disintegrating agents, pigments, binders, lubricants, glidants, flavorants, and so forth may be used for customary purposes and in typical amounts without adversely affecting the properties of the compositions. These excipients may be utilized after the drug/polymer composition has been formed, in order to formulate the composition into tablets, capsules, suppositories, suspensions, powders for suspension, creams, transdermal patches, depots, and the like.

The compositions of the present invention may be delivered by a wide variety of routes, including, but not limited to, oral, nasal, rectal, vaginal, subcutaneous, intravenous and pulmonary. Generally, the oral route is preferred.

The compositions of the present invention may be formulated in various forms such that they are delivered as a suspension of particles in a liquid vehicle. Such suspensions may be formulated as a liquid or paste at the time of manufacture, or they may be formulated as a dry powder with a liquid, typically water, added at a later time but prior to oral administration. Such powders that are constituted into a suspension are often termed sachets or oral powder for constitution (OPC) formulations. Such dosage forms can be formulated and reconstituted via any known procedure. The simplest approach is to formulate the dosage form as a dry powder that is reconstituted by simply adding water and agitating. Alternatively, the dosage form may be formulated as a liquid and a dry powder that are combined and agitated to form the oral suspension. In yet another embodiment, the dosage form can be formulated as two powders that are reconstituted by first adding water to one powder to form a solution to which the second powder is combined with agitation to form the suspension.

Generally, it is preferred that the composition of drug be formulated for long-term storage In the dry state as this promotes the chemical and physical stability of the drug. Since conversion of amorphous drug In the composition to the crystalline state is related to the relative values of (1) the T_{g} of the composition and (2) T_{storage}, the drug in the solid compositions of the present invention may tend to remain in the amorphous state for forger periods when stored at relatively low temperatures and low relative humidities. In addition, packaging of such compositions so as to prevent absorption of water or inclusion of a water absorbing material such as a desiccant to also prevent or retard water absorption can lead to a higher T_{g} for the composition during storage, thereby helping to retain the amorphous state. Likewise, storage at lower temperatures can also Improve the retention of the amorphous state.

### EXAMPLES

### Reference, Example 1

A solid composition was formed by a spray-drying process as follows. The composition consisted of 50 wt% of the low-solubility HIV protease inhibitor N-(1,1-dimethylethyl) decahydro-2- [(2R,3R)-2-hydroxy-3-[(3-hydroxy-2-methylbenzoyl)amino]-4-(phenylthlo)butyl]-3-isoquinolinecarboxamide (3s, 4aS, 8aS)-monomethanesulfonate, also known as nelfinavir mesylate, or VIRACEPT® ("Drug 1") (solubility approximately 20 µg/mL in PBS, pH 6.5, T_{g} of 119°C at less than 5% RH), 30 wt% of poloxamer 407 (PLURONIC F127, available from BASF Corporation, Mount Olive, New Jersey), and 20 wt% of the stabilizing polymer hydroxypropyl methylcellulose (HPMC E3 Prem LV, METHOCEL®, available from Dow Chemical Co., Midland, Michigan, having a T_{g} of about 150°C at less than 5% relative humidity). A spray solution was formed containing 2.5 wt% Viracept, 1.5 wt% Pluronic F127, 1.0 wt% HPMC, 85.5 wt% methanol, and 9.5 wt% water. The solutions was mixed until the polymers and drug had all dissolved. The spray solution was pumped using a highpressure pump to a spray drier (a Niro type XP Portable Spray-Dryer with a Liquid-Feed Process Vessel CPSD-1")), equipped with a pressure nozzle (model Wag-126. from Delavan LTV). The PSD-1 was equipped with a 5-foot S-inch chamber extension. The chamber extension was added to the spray dryer to increase the vertical length of the dryer. The added length increased the residence time within the dryer, which allowed the product to dry before reaching the angled section of the spray dryer. The spray drier was also equipped with a 316 SS circular diffuser plate with 1/16-inch drilled holes, having a 1% open area. This small open area directed the flow of the drying gas to minimize product recirculation within the spray dryer. The nozzle sat flush with the diffuser plate during operation. The spray solution was delivered to the nozzle at about 120 g/min at a pressure of 230 psig. A pulsation dampener was used to minimize pulsation at the nozzle. Drying gas (e.g., nitrogen) was introduced through the diffuser plate at a flow rate of about 2000 g/min, and an inlet temperature of about 175°C. The evaporated solvent and drying gas exited the spray drier at a temperature of about 45°C. The spray-dried composition was collected in a cyclone, and contained 50 wt% Drug 1, 30 wt% Pluronic, and 20 wt% HPMC; thus, the mass ratio of poloxamer to stabilizing polymer was 1.5 (30 wt% ÷ 20 wt%). The T_{g} of the resulting composition was determined by DSC to be about 107°C at less than 5% RH.

The so-formed solid composition was examined for drug crystallinity before and after storage for 3 weeks storage at 40°C and 75% RH by powder x-ray diffraction (PXRD) using a Bruker AXS D8 Advance diffractometer. Samples (approximately 100 mg) were packed in Lucite sample cups fitted with Si(511) plates as the bottom of the cup to give no background signal. Samples were spun in theϕ) plane at a rate of 30 rpm to minimize crystal orientation effects. The x-ray source (KCu_{α}, λ= 1.54 A) was operated at a voltage of 45 kV and a current of 40 mA. Data for each sample were collected over a period of 27 minutes in continuous detector scan mode at a scan speed of 1.8 seconds/step and a step size of 0.04°/step. Diffractograms were collected over the 2θ range of 4° to 30°.

The solid composition of Example 1 before and after storage for 3 weeks at 40°C and 75% RH exhibited diffraction patterns indicating that the drug in the composition was almost completely amorphous, showing none of the sharp peaks that are characteristic of crystalline drug. This demonstrates that the amorphous form of the drug is stable in the composition after storage at 40°C and 75% RH for 3 weeks.

The solid composition of Example 1 was evaluated in an *in vitro* dissolution test as follows. For each test, the composition was added to microcentrifuge tubes in duplicate. A 4.21 mg sample of the composition was added to each tube for a total concentration of 1000 µg/mL drug, if all of the drug had dissolved. The tubes were placed in a 37°C temperature-controlled chamber, and 1.8 mL PBS containing 0.5 wt% sodium taurocholic acid and 1-palmitoyl-2-oleyl-sn-glycero-3-phosphocholine (NaTC/POPC, with a 4/1 weight ratio) at pH 6.5 and 290mOsm/kg. (model fasted duodenal solution, "MFDS") was added to each respective tube. The samples were quickly mixed using a vortex mixer for about 60 seconds. The samples were centrifuged at 13,000 G at 37°C for 1 minute. The resulting supernatant solution was then sampled and diluted 1:6 (by volume) with methanol and then analyzed by high-performance liquid chromatography (HPLC). The contents of each respective tube were mixed on the vortex mixer and allowed to stand undisturbed at 37°C until the next sample was taken. Samples were collected at 4, 10, 20, 40, 90, and 1200 minutes.

As a control (C1), crystalline Drug 1 alone was added 1.8 mL of MFDS, at a sufficient amount so that the concentration of drug would have been 1000 µg/mL, if all of the drug had dissolved.

As a second control (C2), a composition was formed that was similar to Example 1 except that it did not contain the poloxamer. Control C2 consisted of 50 wt% Drug 1 and 50 wt% HPMC. The composition was formed by first forming a spray solution consisting of 292 mg Drug 1 and 250 mg HPMC in 58.7 gm of a 50.7:8 w/w mixture of acetone:water. This solution was pumped into a "mini" spray-drying apparatus via a Cole Parmer 74900 series rate-controlling syringe pump at a rate of 70 ml/hr. The spray solution was atomized through a Spraying Systems Co. two-fluid nozzle, Model No. SU1A, using a heated stream of nitrogen at a flow rate of 1 SCFM. The spray solution was sprayed into an 11-cm diameter stainless steel chamber. The heated gas entered the chamber at an inlet temperature of 110°C and exited at ambient temperature. The resulting solid composition was collected on filter paper, dried under vacuum, and stored in a desiccator. This composition was tested in a dissolution test using the procedures described above except that a sufficient amount of the composition was added so that the concentration if all of the drug had dissolved would have been 3000 µg/mL.

The results from the dissolution tests of Example 1, and controls C1 and C2 are shown in Table 2.

**Table 2**

| Example | Time (min) | Drug 1 Concentration (µg/mL) | AUC (min*µg/mL) |
|---|---|---|---|
| 1 | 0 | 0 | 0 |
| | 4 | 330 | 660 |
| | 10 | 368 | 2,760 |
| | 20 | 377 | 6,490 |
| | 40 | 382 | 14,100 |
| | 90 | 385 | 33,300 |
| | 1200 | 390 | 463,800 |
| C1 | 0 | 0 | 0 |
| | 4 | 80 | 160 |
| | 10 | 98 | 700 |
| | 20 | 102 | 1, 700 |
| | 40 | 108 | 3,800 |
| | 90 | 118 | 9,500 |
| | 1200 | 310 | 247,000 |
| C2 | 0 | 0 | 0 |
| | 4 | 118 | 235 |
| | 10 | 123 | 960 |
| | 20 | 122 | 2,180 |
| | 40 | 117 | 4,570 |
| | 90 | 124 | 10,600 |
| | 1200 | 116 | 143,500 |

The concentrations of drug obtained in these samples were used to determine the maximum concentration of drug ("MDC₉₀") and the area under the concentration-versus-time curve ("AUC₉₀") during the initial ninety minutes. The results are shown in Table 3.

**Table 3**

| Example | Drug1 1 Conc. in Composition (wt%) | Media | Dose (µg/mL) | MDC₉₀ (µg/mL) | AUC₉₀ (min*µg/mL) |
|---|---|---|---|---|---|
| 1 | 50 | MFDS | 1000 | 385 | 33,300 |
| C1 (crystalline Drug 1) | -- | MFDS | 1000 | 118 | 9,500 |
| C2 (No poloxamer) | 50 | MFDS | 3000 | 124 | 10,600 |

The data show that the composition of the invention provided concentration-enhancement relative to both control compositions C1 and C2. The MDC₉₀ for Example 1 was 3.3-fold that of the control C1, and the AUC₉₀ for Example 1 was 3.5-fold that of the control C1. The MDC₉₀ for Example 1 was 3.1-fold that of Control C2, and the AUC₉₀ for Example 1 was 3.1-fold that of control C2.

The stability of the solid composition of Example 1 was evaluated in an accelerated storage test. The composition was stored under elevated temperature and humidity conditions to increase the rate of physical changes occurring in the material in order to simulate a longer storage interval in a typical storage environment. A sample of the composition was stored for 3 weeks at 40°C/75% RH. Following storage the composition was tested *in vitro,* as described above, to demonstrate stable concentration-enhancement of Drug 1. The results are shown in Table 4.

**Table 4**

| Example | Time (min) | Drug 1 Concentration (µg/mL) | AUC (min*µg/mL) |
|---|---|---|---|
| 1 stored 3 weeks @ 40°C/75% RH | 0 | 0 | 0 |
| | 4 | 371 | 740 |
| | 10 | 378 | 2,990 |
| | 20 | 389 | 6,830 |
| | 40 | 373 | 14,400 |
| | 90 | 389 | 33,500 |
| | 1200 | 416 | 480,300 |

The concentrations of drug obtained in these samples were used to determine the MDC₉₀ and the AUC₉₀ during the initial ninety minutes. The results are shown in Table 5. The dissolution test results prior to storage are shown again for comparison.

**Table 5**

| Example | Drug Concentration in Composition (wt%A) | Dose (µg/mL) | MDC₉₀ (µg/mL) | AUC₉₀ (min*µg/mL) |
|---|---|---|---|---|
| 1 initial | 50 | 1000 | 385 | 33,300 |
| 1 stored 3 weeks @ 40°C/75% RH | 50 | 1000 | 389 | 33,500 |

The data show that the composition of the invention provided stable concentration-enhancement after storage for 3 weeks at 40°C/75% RH.

### Examples 2-4

Solid compositions containing Drug 1 were prepared using various amounts of poloxamer 407 and the stabilizing polymers hydroxypropyl methyl cellulose and hydroxypropyl methyl cellulose acetate succinate as described below. Table6 gives the compositions of reference Example 2 and Examples 3 and 4; the composition of reference Example 1 is included for comparison. The Tg of each composition, measured at a relative humidity of less than 10%, is also included in Table 6.

A control composition C3 was made with the polymer hydroxypropyl cellulose.

**Table 6**

| Exp. No. | Drug 1 (wt%) | PLURONIC F127 (wt%) | Stabilizing Polymer* | Stabilizing Polymer (wt%) | Tg of the Composition at <10% RH (°C) |
|---|---|---|---|---|---|
| 1** | 50 | 30 | HPMC E3 Prem LV | 20 | 107 |
| 2** | 35 | 35 | HPMC E3 Prem LV | 30 | 97 |
| 3 | 42.5 | 42.5 | HPMCAS-MF | 15 | 110 |
| 4 | 45 | 45 | HPMCAS-MF | 10 | 110 |
| C3 | 45 | 45 | HPC | 10 | 101 |

| | | | | | |
|---|---|---|---|---|---|
| * HPMCAS-MF = hydroxypropyl methyl cellulose acetate succinate, medium fine grade (Shin Etsu, Tokyo, Japan), having a T_{g} of about 118°C at less than 5% RH. HPC = hydroxypropyl cellulose (Sigma-Aldrich, Inc., #43,500-7), having a T_{g} of about 125°C at less than 5% RH. ** Reference examples | | | | | |

The solid composition of Example 2 was prepared using the spray drying process described for Example 1, except that the spray solution consisted of 7 gm Drug 1, 7 g PLURONIC F127, 6 g HPMC E3 Prem LV, 342 g methanol, and 38 g water.

The solid compositions of Examples 3, 4, and C3 were spray-dried using a "mini" spray drier as follows. Drug 1, PLURONIC F127, and the stabilizing polymer were dissolved in 24 g of acetone. For Control C3, the total solids concentration in the spray solution was 2 wt%. For examples 3 and 4, the total solids concentration in the spray solutions were 4 wt%. Each solution was pumped into a "mini" spray-drying apparatus via a Cole Parmer 74900 series rate-controlling syringe pump at a rate of 30 ml/hr. The spray solution was atomized through a Spraying Systems Co. two-fluid nozzle, Model No. SU1A, using a heated stream of nitrogen at a flow rate of 1 SCFM. The spray solution was sprayed into an 11-cm diameter stainless steel chamber. The heated gas entered the chamber at an inlet temperature of 80°C and exited at ambient temperature. The resulting solid composition was collected on filter paper, dried under vacuum, and stored in a desiccator.

The solid compositions of Examples 2 to 4 and C3 were tested *in vitro* to demonstrate concentration-enhancement of Drug 1. For each test, a sufficient amount of composition was added to each tube for a total concentration of 1000 µg/mL drug, if all of the drug had dissolved. The tubes were placed in a 37°C temperature-controlled chamber, and 1.8 mL PBS was added. Tests were performed as described above, and the results are shown in Table 7.

As a control (C4), crystalline Drug 1 alone was tested in PBS and a sufficient amount of material was added so that the concentration of drug would have been 1000 µg/mL, if all of the drug had dissolved.

**Table 7**

| Example | Time (min) | Drug 1 Concentration (µg/mL) | AUC (min*µg/mL) |
|---|---|---|---|
| 2 | | 0 | 0 |
| | 4 | 376 | 750 |
| | 10 | 399 | 3,080 |
| | 20 | 399 | 7,070 |
| | 40 | 437 | 15,400 |
| | 90 | 419 | 36,800 |
| | 1200 | 543 | 570,500 |
| 4 | 0 | 0 | 0 |
| | 4 | 249 | 500 |
| | 10 | 260 | 2,020 |
| | 20 | 283 | 4,740 |
| | 40 | 309 | 10,700 |
| | 90 | 331 | 26,700 |
| | 1200 | 386 | 424,800 |
| 5 | 0 | 0 | 0 |
| | 4 | 177 | 350 |
| | 10 | 190 | 1,450 |
| | 20 | 215 | 3,480 |
| | 40 | 238 | 8,000 |
| | 90 | 266 | 20,600 |
| | 1200 | 325 | 348,800 |
| C3 | 0 | 0 | 0 |
| | 4 | 84 | 170 |
| | 10 | 65 | 610 |
| | 20 | 61 | 1,240 |
| | 40 | 35 | 2,200 |
| | 90 | 19 | 3,550 |
| | 1200 | 13 | 21,300 |
| C4 | 0 | 0 | 0 |
| | 4 | 5 | 11 |
| | 10 | 6 | 46 |
| | 20 | 6 | 106 |
| | 40 | 2 | 180 |
| | 90 | 2 | 285 |
| | 1200 | 20 | 12,730 |

The concentrations of drug obtained in these samples were used to determine the MDC₉₀ and the AUC₉₀ during the initial ninety minutes. The results are shown in Table 8.

**Table 8**

| Example | Drug 1 Conc. in Composition (wt%A) | Stabilizing Polymer | MDC₉₀ (µg/mL) | AUC₉₀ (min*µg/mL) |
|---|---|---|---|---|
| 2 | 35 | HPMC | 437 | 36,800 |
| 3 | 42.5 | HPMCAS-MF | 331 | 26,700 |
| 4 | 45 | HPMCAS-MF | 266 | 20,600 |
| C3 | 45 | HPC | 84 | 3,550 |
| C4 | -- | none | 6 | 285 |

The data show that the compositions of the invention provided concentration-enhancement over that of crystalline drug alone (C4). The MDC₉₀ for Examples 2 to 4 was 44-to 73-fold that of the crystalline control, and the AUC₉₀ for Examples 2 to 4 was 72- to 129-fold that of the crystalline control. In addition, Examples 2 to 4 provided concentration enhancement relative to a different cellulosic polymer, hydroxypropyl cellulose.

### Example 5

A solid composition was formed containing the low-solubility cholesteryl ester transfer protein inhibitor [2R,4S] 4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester, also known as torcetrapib ("Drug 2"), having a solubility of less than 1 µg/mL in PBS, pH 6.5, and a T_{g} of about 30°C at less than 5% RH. The composition consisted of 25 wt% Drug 2, 50 wt% PLURONIC F127, and 25 wt% HPMCAS-MF; thus, the mass ratio of poloxamer to stabilizing polymer was 2 (50 wt% ÷ 25 wt%). The composition was formed using a spray drying process described for Examples 2 to 4, except that the spray solution consisted of 0.8 wt% Drug 2, 1.6 wt% Pluronic F127, 0.8 wt% HPMCAS-MF, and 96.8 wt% acetone; the solution feed rate was 60 mUhr, and the drying gas inlet temperature was 85°C.

The so-formed solid composition was tested *in vitro* to demonstrate concentration-enhancement of Drug 2. A sufficient amount of the composition was added to each tube (in duplicate) for a total concentration of 1000 µg/mL drug, if all of the drug had dissolved. The tubes were placed in a 37°C temperature-controlled chamber, and 1.8 mL PBS was added. Tests were performed as described above, and the results are shown in Table 9.

Control 5 (C5) consisted of crystalline Drug 2 alone, tested in PBS, and a sufficient amount of material was added so that the concentration of drug would have been 1000 µg/mL, if all of the drug had dissolved.

**Table 9**

| Example | Time (min) | Drug 2 Concentration (µg/mL) | AUC (min*µg/mL) |
|---|---|---|---|
| 5 | 0 | 0 | 0 |
| | 4 | 455 | 910 |
| | 10 | 423 | 3,550 |
| | 20 | 399 | 7,660 |
| | 40 | 336 | 15,000 |
| | 90 | 276 | 30,300 |
| | 1200 | 85 | 230,600 |
| C5 | 0 | <1 | <100 |
| | 4 | <1 | <100 |
| | 10 | <1 | <100 |
| | 20 | <1 | <100 |
| | 40 | <1 | <100 |
| | 90 | <1 | <100 |
| | 1200 | <1 | <100 |

The concentrations of drug obtained in these samples were used to determine the MDC₉₀ and the AUC₉₀ during the initial ninety minutes. The results are shown in Table 10.

**Table 10**

| Example | Drug 2 Conc. in Composition (wt%A) | Dose (µg/mL) | MDC₉₀ (µg/mL) | AUC₉₀ (min*µg/mL) |
|---|---|---|---|---|
| 5 | 25 | 1000 | 455 | 30,300 |
| C4 | -- | 1000 | <1 | <100 |

The data show that the solid composition of the invention provided concentration-enhancement over that of crystalline drug alone (C4). The MDC₉₀ for the solid composition of Example 5 was greater than 455-fold that of the control, while the AUC₉₀ for the composition of Example 5 was greater than 303-fold that of the control.

### Example 6

A solid composition was formed containing 40 wt% Drug 2, 30 wt% PLURONIC F127, and 30 wt% HPMCAS-MF; thus, the mass ratio of poloxamer to stabilizing polymer was 1 (30 wt% ÷ 30 wt%). A rotoevaporation process was used to form the solid composition as follows. First, 0.4 g of Drug 2, 0.3 g of PLURONIC F127, and 0.3 g of HPMCAS-MF were added to 15 mL methanol in a round-bottom flask, and stirred for 2 hours at room temperature. Next, the methanol was removed from the solution under vacuum (less than about 0.1 atm), while rotating the flask at 120 rpm in a 30°C bath. The resulting material was then removed from the flask, chilled in liquid nitrogen, and ground with a mortar and pestle.

The so-formed solid composition was tested *in vitro* to demonstrate concentration-enhancement of Drug 2. A sufficient amount of the composition was added to each tube (in duplicate) for a total concentration of 1000 µg/mL drug, if all of the drug had dissolved. The tubes were placed in a 37°C temperature-controlled chamber, and 1.8 mL PBS was added. Tests were performed as described above, and the results are shown in Table 11.

**Table 11**

| Example | Time (min) | Drug 2 Concentration (µg/mL) | AUC (min* µg/mL) |
|---|---|---|---|
| 6 | 0 | 0 | 0 |
| | 4 | 267 | 500 |
| | 10 | 260 | 2100 |
| | 20 | 212 | 4500 |
| | 40 | 177 | 8400 |
| | 90 | 139 | 16,300 |
| | 1200 | 76 | 135,200 |

The concentrations of drug obtained in these samples were used to determine the MDC₉₀ and the AUC₉₀ during the initial ninety minutes. The results are shown in Table 12. Control 5 (C5), consisting of crystalline Drug 2 alone, is shown again for comparison.

**Table 12**

| Example | Drug 2 Conc. in Composition (wt%A) | Dose (µg/mL) | MDC₉₀ (µg/mL) | AUC₉₀ (min*µg/mL). |
|---|---|---|---|---|
| 6 | 40 | 1000 | 267 | 16,300 |
| C5 | -- | 1000 | <1 | <100 |

The data show that the solid composition of the invention provided concentration-enhancement over that of crystalline drug alone (C5). The MDC₉₀ for the solid composition of Example 6 was greater than 267-fold that of the control, while the AUC₉₀ for the composition of Example 6 was greater than 163-fold that of the control.

The stability of the solid composition of Example 6 was evaluated in a storage test. The composition was stored for 3 weeks at 25°C/10% RH, and drug crystallinity was evaluated before and after storage. Samples were examined using PXRD, as described for Example 1 above. The solid composition of Example 6 before and after storage for 3 weeks at 25°C and 10% RH exhibited diffraction patterns indicating that the drug in the composition was almost completely amorphous, showing none of the sharp peaks that are characteristic of crystalline drug.

### Example 7

A solid composition was formed containing 25 wt% Drug 2, 65 wt% HPMCAS-HF, and 10 wt PLURONIC F127. A rotoevaporation process was used to form the solid composition, as described for Example 6.

The so-formed solid composition was tested *in vitro* to demonstrate concentration-enhancement of Drug 2. A sufficient amount of the composition was added to each tube (in duplicate) for a total concentration of 1000 µg/mL drug, if all of the drug had dissolved. The tubes were placed in a 37°C temperature-controlled chamber, and 1.8 mL PBS was added. Tests were performed as described above, and the results are shown in Table 13.

As a control (C6), a composition consisting of 25 wt% Drug 2 and 75 wt% HPMCAS-HF was formed using the rotoevaporation process described for Example 6. The composition was tested *in vitro,* and a sufficient amount of material was added to PBS so that the concentration of drug would have been 1000 µg/mL, if all of the drug had dissolved.

**Table 13**

| Example | Time (min) | Drug 2 Concentration (µg/mL) | AUC (min*µg/mL) |
|---|---|---|---|
| 7 | 0 | 0 | 0 |
| | 4 | 7 | <100 |
| | 10 | 45 | 200 |
| | 20 | 117 | 1000 |
| | 40 | 167 | 3800 |
| | 90 | 278 | 15,000 |
| | 1200 | 508 | 450,700 |
| C6 | 0 | 0 | 0 |
| | 4 | 0 | <100 |
| | 10 | 0 | <100 |
| | 20 | 3 | <100 |
| | 40 | 4 | 100 |
| | 90 | 5 | 300 |
| | 1200 | 318 | 179,700 |

The concentrations of drug obtained in these samples were used to determine the MDC₉₀ and the AUC₉₀ during the initial ninety minutes. The results are shown in Table 10. Also shown in Table 10 is the concentration of drug at 1200 minutes (C₁₂₀₀), Control 5 (C5), consisting of crystalline Drug 2 alone, is shown- again for comparison.

**Table 10**

| Example | Drug 2 Conc. in Composition (wt%A) | Dose (µg/mL) | MDC₉₀ (µg/mL) | AUC₉₀ (min*µg/mL) | C₁₂₀₀ (µg/mL) |
|---|---|---|---|---|---|
| 7 | 25 | 1000 | 278 | 15,000 | 508/ |
| C6 | 25 | 1000 | 5 | 300 | 318 |
| C5 | -- | 1000 | <1 | <100 | <1 |

The data show that the solid composition of the invention provided concentration-enhancement over that of the control composition without the poloxamer (C6), as well as concentration-enhancement relative to crystalline drug alone (C5). The MDC₉₀ for the solid composition of Example 7 was 56-fold that of control C6, while the AUC₉₀ for the composition of Example 7 was 50-fold that of control C6. The concentration enhancement of Drug 2 provided by the composition of Example 7 relative to control example C6 was due to an increased rate of dissolution, as evidenced by the higher dissolved drug concentration after 1200 minutes (C₁₂₀₀).

## Claims

1. A solid composition comprising a plurality of particles, said particles comprising:
(a) at least 5 wt% of a low-solubility drug having a minimum aqueous solubility at pH of 1-8 of less than 0.5 mg/ml, wherein at least 75 wt% said drug is amorphous;
(b) at least 5 wt% of a poloxamer; and
(c) a stabilizing polymer selected from the group consisting of hydroxypropyl methyl cellulose acetate succinate and carboxymethyl ethyl cellulose.

2. The solid composition of claim 1 wherein said particles have a lowest , glass transition temperature of at least 40°C at a relative humidity of less than 10%.

3. The solid composition of claim 2 wherein the lowest glass-transition temperature of said particles is at least 45°C at a relative humidity of less than 5%.

4. The solid composition of claim 2 wherein the lowest glass-transition temperature of said particles is at least 50°C at a relative humidity of less than 5%.

5. The solid composition of any one of claims 1-4 wherein said drug has a glass-transition temperature of at least 20°C at a relative humidity of less than 5%.

6. The solid composition of any one of claims 1 -5 wherein said drug has a glass-transition temperature of at least 30°C at a relative humidity of less than 5%.

7. The solid composition of any one of claims 1-6 wherein said poloxamer is selected from the group consisting of poloxamer 188, poloxamer 237, poloxamer 338, and poloxamer 407.

8. The solid composition of anyone of claims 1-7 wherein said drug is selected from the group consisting of antihypertensives, antianxiety agents, anticlotting agents, anticonvulsants, blood glucose-lowering agents, decongestants, antihistamines, antitussives, antineoplastics, beta blockers, anti-inflammatories, antipsychotic agents, cognitive enhancers, cholesterol-reducing agents, anti-atherosclerotic agents, antiobesity agents, autoimmune disorder agents, anti-impotence agents, antibacterial and antifungal agents, hypnotic agents, anti-Parkinsonism agents, anti-Alzheimer's disease agents, antibiotics, anti-depressants, antiviral agents, glycogen phosphorylase inhibitors, microsomal triglyceride transfer protein inhibitors, and cholesteryl ester transfer protein Inhibitors.

9. The solid composition of any one of claims 1-8 wherein said drug is a hydrophobic drug.

10. The solid composition of any one of claims 1-9 wherein said drug is selected from the group consisting of N-(1,1-dimethylethyl) decahydro-2-[(2R,3R)-2-hydroxy-[(3-hydroxy-2-methylbenzoyl)amino]-4-(phenylthio)butyl]-3-isoquinolinecarboxamide (3s, 4aS, 8aS)-monomethanesulfonate, [2R,4S] 4-[(3,5-bis-trifluoromethyl-benzyl)methoxycarbonyl-amino]-2-athyl-6-trinuoromethyl-3,4-dihydro-2H-quinoline-1-carboxylic add ethyl ester, or pharmaceutically acceptable forms thereof.

11. The solid composition of any one of claims 1-10 wherein said composition is made by a solvent-based process.

12. The solid composition of claim 11 wherein said solvent-based process is spraydrying.

## Patentansprüche

1. Feste Zusammensetzung, die eine Vielzahl von Partikeln umfasst, wobei die Partikel umfassen:
(a) wenigstens 5 Gew.-% eines Wirkstoffs mit geringer Löslichkeit, der eine Mindestlöslichkeit in Wasser bei pH 1-8 von weniger als 0,5 mg/ml hat, wobei wenigstens 75 Gew.-% des Wirkstoffs amorph sind;
(b) wenigstens 5 Gew.-% eines Poloxamers und
(c) ein stabilisierendes Polymer, ausgewählt aus der Gruppe, bestehend aus Hydroxypropylmethylcelluloseacetatsuccinat und Carboxymethyethylcellulose.

2. Feste Zusammensetzung nach Anspruch 1, wobei die Partikel eine niedrigste Glasübergangstemperatur von wenigstens 40°C bei einer relativen Feuchtigkeit von weniger als 10% haben.

3. Feste Zusammensetzung nach Anspruch 2, wobei die niedrigste Glasübergangstemperatur der Partikel wenigstens 45°C bei einer relativen Feuchtigkeit von weniger als 5% ist.

4. Feste Zusammensetzung nach Anspruch 2, wobei die niedrigste Glasübergangstemperatur der Partikel wenigstens 50°C bei einer relativen Feuchtigkeit von weniger als 5% ist.

5. Feste Zusammensetzung nach einem der Ansprüche 1 bis 4, wobei der Wirkstoff eine Glasübergangstemperatur von wenigstens 20°C bei einer relativen Feuchtigkeit von weniger als 5% hat.

6. Feste Zusammensetzung nach einem der Ansprüche 1 bis 5, wobei der Wirkstoff eine Glasübergangstemperatur von wenigstens 30°C bei einer relativen Feuchtigkeit von weniger als 5% hat.

7. Feste Zusammensetzung nach einem der Ansprüche 1 bis 6, wobei das Poloxamer ausgewählt ist aus der Gruppe, bestehend aus Poloxamer 188, Poloxamer 237, Poloxamer 338 und Poloxamer 407.

8. Feste Zusammensetzung nach einem der Ansprüche 1 bis 7, wobei der Wirkstoff ausgewählt ist aus der Gruppe, bestehend aus Antihypertensiva, Mitteln gegen Angst, Mitteln gegen Blutgerinnung, Antikonvulsiva, Blutglukose-senkenden Mitteln, Entstauungsmitteln, Antihistaminen, Antitussiva, antineoplastischen Mitteln, Betablockern, antiinflammatorischen Mitteln, Antipsychotika, Wahrnehmungsverstärkern, cholesterinsenkenden Mitteln, Mitteln gegen Atherosklerose, Mitteln gegen Adipositas, Mitteln gegen Autoimmunstörungen, Mitteln gegen Impotenz, antibakteriellen und antifungalen Mitteln, Hypnotika, Mitteln gegen Parkinsonismus, Mitteln gegen die Alzheimer'sche Krankheit, Antibiotika, Antidepressiva, antiviralen Mitteln, Glycogenphosphorylaseinhibitoren, Inhibitoren des mikrosomalen Triglyceridtransferproteins und Inhibitoren des Cholesterylestertransferproteins.

9. Feste Zusammensetzung nach einem der Ansprüche 1 bis 8, wobei der Wirkstoff ein hydrophober Wirkstoff ist.

10. Feste Zusammensetzung nach einem der Ansprüche 1 bis 9, wobei der Wirkstoff ausgewählt ist aus der Gruppe, bestehend aus N-(1,1-Dimethylethyl)decahydro-2-[(2R,3R)-2-hydroxy-3-[(3-hydroxy-2-methylbenzoyl)amino]-4-(phenylthio)butyl]-3-isochinolincarboxamid(3s,4aS,8aS)-monomethansulfonat, [2R,4S]-4-[(3,5-Bis-trifluormethylbenzyl)-methoxycarbonyl-amino]-2-ethyl-6-trifluormethyl-3,4-dihydro-2H-chinolin-1-carbonsäureethylester oder pharmazeutisch verträglichen Formen davon.

11. Feste Zusammensetzung nach einem der Ansprüche 1 bis 10, wobei die Zusammensetzung durch ein Verfahren auf Lösungsmittelbasis hergestellt ist.

12. Feste Zusammensetzung nach Anspruch 11, wobei das Verfahren auf Lösungsmittelbasis Sprühtrocknung ist.

## Revendications

1. Composition solide comprenant une multitude de particules, dans laquelle les particules comprennent:
a) au moins 5% en poids d'un principe actif à solubilité faible ayant une valeur minimale de solubilité dans l'eau à pH 1-8 inférieure à 0,5 mg/ml, 75% en poids de principe actif étant amorphe ;
b) au moins 5% en poids d'un poloxamère, et
c) un polymère stabilisant choisi parmi le groupe consistant en acétate-succinate d'hydroxypropylméthylcellulose et carboxyméthyl-cellulose.

2. Composition solide selon la revendication 1, dans laquelle les particules ont la plus basse température de transition vitreuse d'au moins 40°C dans une humidité relative inférieure à 10%.

3. Composition solide selon la revendication 2, dans laquelle la plus basse température de transition vitreuse des particules est au moins 45°C dans une humidité relative inférieure à 5%.

4. Composition solide selon la revendication 2, dans laquelle la plus basse température de transition vitreuse des particules est au moins 50°C dans une humidité relative inférieure à 5%.

5. Composition solide selon l'une quelconque des revendications 1 à 4, dans laquelle ledit principe actif a une température de transition vitreuse d'au moins 20°C dans une humidité relative inférieure à 5%.

6. Composition solide selon l'une quelconque des revendications 1 à 5, dans laquelle le principe actif a une température de transition vitreuse d'au moins 30°C dans une humidité relative inférieure à 5%.

7. Composition solide selon l'une quelconque des revendications 1 à 6, dans laquelle le poloxamère est choisi parmi le groupe consistant en poloxamère 188, poloxamère 237, poloxamère 338 et poloxamère 407.

8. Composition solide selon l'une quelconque des revendications 1 à 7, dans laquelle le principe actif est choisi parmi le groupe consistant en antihypertenseurs, agents contre l'anxiété, anticoagulants, anticonvulsivants, agents destinés à réduire le taux de glucose dans le sang, agents décongestionnants, antihistaminiques, antitussifs, agents antinéoplasiques, bêtabloquants, anti-inflammatoires, antipsychotiques, agents destinés à améliorer des fonctions cognitives, agents pour réduire le cholestérol, agents contre l'athérosclérose,agents contre l'obésité, agents contres les maladies auto-immunes, agents contre l'impuissance, agents antibactériens et antifongiques, hypnotiques, agents contre la maladies de Parkinson, agents, contre la maladie d'Alzheimer, antibiotiques, antidépresseurs, agents antiviraux, inhibiteurs de la glycogène phosphorylase, inhibiteurs de la protéine microsomale de transfert des triglycérides et inhibiteurs de la protéine de transfert des ester de cholestérol.

9. Composition solide selon l'une quelconque des revendications 1 à 8, dans laquelle le principe actif est un principe actif hydrophobe.

10. Composition solide selon l'une quelconque des revendications 1 à 9, dans laquelle le principe actif est choisi parmi le groupe consistant en N-(1,1-diméthyléthyl)decahydro-2-[(2R,3R)-2-hydroxy-3-[(3-hydroxy-2-méthylbenzoyl)amino]-4-(phénylthio)butyl]-3-isoquinoléinecarboxamide(3s,4aS,8aS)-monométhanesulfonate, l'ester d'éthyle de l'acide [2R,4S]-4-[(3,5-bis-trifluorométhyl-benzyl)-méthoxycarbonylamino]-2-éthyl-6-trifluorométhyl-3,4-dihydro-2H-quinoléine-1-carboxylique ou formes pharmaceutiquement acceptables de ceux-ci.

11. Composition solide selon l'une quelconque des revendications 1 à 10, la composition étant fabriquée par un procédé à base d'un solvant.

12. Composition solide selon la revendication 11, le procédé à base d'un solvant étant le séchage par atomisation.
